# EUROPÄISCHE PATENTANMELDUNG

(11) **EP 2 682 137 A2**
(43) Veröffentlichungstag der Anmeldung: **08.01.2014**
(21) Anmeldenummer: 13174508.5
(22) Anmeldetag: 01.07.2013
(51) Int. Cl.: A61L 27/42, A61L 27/56

(54) **Synthetisches Knochenersatzmaterial und Verfahren zu seiner Herstellung**

(30) Priorität: 01.07.2012 DE 102012211390
(71) Anmelder: Fraunhofer-Gesellschaft zur Förderung der angewandten Forschung e.V., 80686 München (DE)
(72) Erfinder: Ahlhelm, Matthias, 01309 Dresden (DE); Moritz, Tassilo, 09599 Freiberg (DE)
(74) Vertreter: Rauschenbach, Marion

(57) **Zusammenfassung**

Die Erfindung bezieht sich auf das Gebiet der Werkstoffwissenschaften und betrifft synthetische Knochenersatzmaterialien, wie sie Knochen oder Knochenabschnitte ersetzen können.

Aufgabe der vorliegenden Erfindung ist es daher, ein synthetisches Knochenersatzmaterial anzugeben, welches offenzellig ist und eine gute mechanische Festigkeit aufweist.

Die Aufgabe wird gelöst durch ein synthetisches Knochenersatzmaterial, welches aus überwiegend offenzelligen Netzwerken aus dreidimensional miteinander verbundenen Stegen besteht, die die aus 20 - 40 Vol.-% eines oder mehrerer Calciumphosphate und aus 60 - 80 Vol.-% mindestens eines biokompatiblen keramischen Materials bestehen.

Die Aufgabe wird weiterhin gelöst durch ein Verfahren, bei dem aus Calciumphosphaten und aus einem keramischen Material eine Suspension hergestellt, die Suspension auf eine Unterlage gegeben wird, und die Suspension einem Unterdruck bis mindestens zur Verfestigung des Suspensionsmediums ausgesetzt wird, und anschließend der Grünkörper aus dem verfestigten Suspensions-Netzwerk gesintert wird.

## Beschreibung

Die Erfindung bezieht sich auf die Gebiete der Werkstoffwissenschaften und der Medizin und betrifft synthetische Knochenersatzmaterialien, wie sie beispielsweise in den menschlichen oder tierischen Körper implantiert und dort verbleiben können und dadurch teilweise oder ganz Knochen oder Knochenabschnitte ersetzen können, oder auch als Filtermaterial oder Katalysatorträger in Bioreaktoren und ein Verfahren zu seiner Herstellung.

Knochenersatzmaterialien, die menschliche oder tierische Knochen ganz oder teilweise ersetzen sollen, sollen biokompatibel sein und möglichst vollständig vom menschlichen oder tierischen Körper angenommen werden. "Knochenersatzmaterialien sind Biomaterialien. Biomaterialien werden definiert als nicht körpereigene Substanzen, die nach der Implantation in den menschlichen Organismus dort vorhandene strukturelle Elemente, ganze Gewebe und deren (Teil-) Funktionen ersetzen."(zitiert nach: Rueger J. Knochenersatzmaterial. Heutiger Stand und Ausblick. Orthopäde 1998; 27: 72-79.) Die dazu verwendeten Materialien sollen es dem Zellgewebe gestatten, sich auf dem synthetischen Material anzusiedeln, sich zu teilen und zu differenzieren. Vorteilhafterweise sollen die synthetischen Materialien mit der Zeit vom Körper resorbiert und durch eigenes Zellmaterial ersetzt werden (bioaktiv). Vor allem für den Fall einer langsamen Resorption, muss das synthetische Material eine ausreichend hohe mechanische Festigkeit aufweisen, die dem des natürlichen Knochens möglichst nahe kommt. Bereits 1893 im "Gesetz der Transformation der Knochen" [Julius Wolf: Das Gesetz der Transformation von Knochen, Hirschwald, Berlin, 1892, 1. Auflage 2012] ist festgehalten worden, dass die äußere Form, die innere Struktur und die Festigkeit eines Knochens auf die mechanische Beanspruchung eingestellt sind. Wenn der Knochen nicht eine möglichst physiologische Belastung erfährt, wird seine Form und Struktur verloren gehen [Orthopädie und Unfallchirurgie, Springer-Verlag, Berlin Heidelberg 2011]. Eine zu hohe mechanische Festigkeit oder Beanspruchung durch künstliche Implantate, wie sie beispielsweise von keramischen Materialien, wie ZrO₂-Keramiken bekannt ist, führt daher zu einem verstärkten Abbau des Gewebes um das synthetische Knochenersatzmaterial herum und wird den Aufbau eines körpereigenen Stützapparates nicht fördern.

Zur Lösung dieses Problems sind zahlreiche Lösungen bekannt.

Hydroxylapatit ist ein biokompatibles und vor allem bioaktives Material, welches aufgrund seiner Ähnlichkeit mit dem Material des menschlichen und tierischen Knochens in der Lage ist, direkte Bindungen zu dem natürlichen Knochenersatzmaterial herzustellen [Hench, L.L.: J. Am. Ceram. Soc. 74, 1991, 1487-1510; LeGeros, Z. et al: Encycl. Handbook Biomater. Bioeng. 2, 1995, 1429-1463; Ducheyne, P. et al: Biomaterials 20, 1999, 2287-2303].
Allerdings weist Hydroxylapatit den entscheidenden Nachteil auf, dass es nur eine geringe mechanische Festigkeit besitzt und daher als Dauerimplantat nicht geeignet ist [Cleries et al: Biomaterials, 2, 2000, 1861-1865; C.O. Townley, Bioceramics: Materials and Applications, edited by G. Fishman et al, Westerville, 1995; K. Yamaskita et al: J. Am. Ceram, Soc. 1986; L. Pramatarova et al: Adv. Mater. 7(1), 2005, 469].
Daher wird Hydroxylapatit häufig nur zur Beschichtung künstlicher Knochenstrukturen oder zum Verfüllen größerer Knochendefekte eingesetzt.

Ein weiterer Nachteil von Hydroxylapatit besteht darin, dass seine Resorption durch den Körper verhältnismäßig lange dauert, so dass Hydroxylapatit häufig nur als Granulat zur Verfüllung von Knochendefekten oder als Schichtmaterial zur besseren Anbindung des künstlichen Knochens an das natürliche Gewebe eingesetzt wird und nicht als eigenständiger Implantatwerkstoff.

Zirkoniumoxid und Zirkoniumoxid-verstärktes Aluminiumoxid (ZTA) werden z.B. als synthetische Zahnmaterialien für Brücken und Einzelkronen, für Wurzelstifte oder im Gelenkbereich als Kugel- oder Pfannenmaterial eingesetzt. Diese Materialien bieten eine sehr hohe mechanische Festigkeit zusammen mit bioinerten Eigenschaften [Li, J. et al: J. Mater. Sci.: Mater. Med. 4 (1993), 50; Piconi, C. et al: Biomaterials 20, 1999; Willmann, G. et al: Biomaterials 17, 1996, 2157-2162].
Von Nachteil ist bei diesen Materialien, dass keine Resorption durch den menschlichen oder tierischen Körper erfolgt, da diese Materialien per se nicht bioaktiv sind, sowie dicht gesintert vorliegen und die Zellen nicht in diese Teile hineinwachsen können. Im Hinblick auf das "Gesetz der Transformation von Knochen" eignen sich diese Materialien ebenso nicht als synthetische Knochenersatzmaterialien, da sie zur Zurückbildung des angrenzenden natürlichen Gewebes und Knochens führen.

Ebenfalls bekannt ist der Einsatz von Metallen (z.B. Stahl oder Titanlegierungen) als stabile, gerüstbildende Strukturen für synthetische Knochenstrukturen, die mit biokompatiblen Calciumphosphaten, wie Hydroxylapatit, beschichtet werden, oder die als beispielsweise Stahlschaum mit Knochenzement gefüllt werden, und als Hybridimplantat ihren Einsatz im menschlichen Körper finden können. [International Conference on Cellular Materials, CellMat 2010, Proceedings: held in Dresden, 27-29 Oct 2010, 2010, 219-224].
Der Vorteil dieser Lösung ist, dass das metallische Gerüst eine hohe Festigkeit mit sich bringt und zusätzlich eine, wenn auch langsame, Resorption des metallischen Materials und die spätere Anbindung an den in den Poren befindlichen Knochenzement (Hydroxylapatit) erfolgt. Allerdings werden hier Metallionen frei, die im Körper diverse Unverträglichkeitsreaktionen, wie beispielsweise Allergien, auslösen können.

Aus der US 2011/0195378 A ist ein bioaktives Bio-Keramik-Komposit-Material bekannt, welches aus einem nahezu inerten bio-keramischen Pulver und einem bioaktiven keramischen Pulver besteht, wobei das bioaktive keramische Pulver gut verteilt über dem nahezu inerten bio-keramischen Pulver über eine Sinterung vorliegt. Bei diesem Komposit-Material werden als bioinerte Pulver Zirkoniumoxid, Aluminiumoxid, Kohlenstoff-basierte Materialien oder Yttrium-stabilisiertes Zirkoniumoxid mit geringem Anteil an Übergangs- oder Seltenerdmetallen verwendet. Als bioaktive Pulver werden oberflächenbioaktive Materialien und vollständig resorbierbare Materialien, wie Hydroxylapatit, bioaktive Gläser, Glaskeramiken, Tricalciumphosphat, Calciumsulfat und Biokeramiken verwendet. Die angegebenen Herstellungsverfahren, wie Spritzgießen, Schlickergießen und Druckgießen mit anschließender Sinterung, legen nahe, dass die erhaltenen Komposit-Materialien dichte Bauteile sind. Dies umso mehr, da diese Bauteile als Knie- oder Hüftendoprothesen oder als Zahnersatz eingesetzt werden sollen.
Nachteilig bei der Herstellung dieses Materials ist, dass die Porosität nicht von vornherein im Material einstellbar ist, sondern erst über die Porenbildung durch Resorption des bioaktiven Materials erreicht wird.

Aus der KR 100 294 008 A ist ein Herstellungsverfahren für ein keramisches Komposit-Implantatmaterial bekannt, welches eine verbesserte Bioverträglichkeit und eine gesteigerte Härte aufweist. Dieses Material wird aus einem metallorganischen Al-Precursor über Hydrolyse hergestellt und mit Zirkoniumoxidpulver beschichtet. Das auf diese Weise erzeugte Pulver wird mit Hydroxylapatit vermischt und bei 1250 °C in Grafitformen in inerter Atmosphäre unter 20 MPa Ar-Gasdruck verdichtet. Ziel einer solchen Behandlung ist die Herstellung einer möglichst dichten Keramik.

Aus der BR 0105243 A ist ebenfalls ein Verfahren zur Herstellung eines KompositMaterials auf Basis von teilstabilisiertem Zirkoniumoxid und Hydroxylapatit bekannt, bei dem eine Strukturkeramik mit hoher Homogenität erzeugt wird. Diese Strukturkeramik findet für orthopädische und dentale Implantate Anwendung. Als Einsatzbereiche werden Femur, Hüfte, Knie, Zahnschmelz und Dentin genannt. Auch aufgrund der Anwendungsangaben ist davon auszugehen, dass dichte Bauteile hergestellt werden.

Ebenfalls ist aus der KR 2009 0000894 A ein Zirkoniumoxid-Hydroxylapatit-Komposit bekannt, bei dem die Zirkoniumoxidphase gleichmäßig im Hydroxylapatit verteilt vorliegt. Dafür wird ein Hydroxylapatit-Zirkoniumoxid-Kompositpulver hergestellt, welches anschließend verdichtet und gesintert wird.

Verschiedene Mischoxide werden dafür genutzt, einen Hydroxylapatit-Zirkoniumoxid-Verbundwerkstoff herzustellen, in welchem die Phasenumwandlung des Hydroxylapatits und des Zirkoniumoxids während der Sinterung unter Atmosphärendruck unterdrückt wird (KR 100788494 A).

Auch wird in der KR 100492270 A ein keramischer Komplex für die Biotransplantation offenbart, der die nachteiligen physikalischen Eigenschaften von Hydroxylapatit durch dessen Verstärkung kompensieren soll. Dieser Komplex besteht aus einer Fluorohydroxylapaptit-Festphasenlösung mit einer Verstärkungsphase, die mindestens eines der Materialien ZrO₂, Al₂O₃, TiO₂, SiC, Si₃N₄ sowie Metallfasern enthält. Der Gehalt der zweiten Phase wird mit 20 - 50 Vol.-% angegeben.

KR 100501674 A offenbart ein Herstellungsverfahren für ein nanoskaliges Hydroxylapatit/Yttrium-stabilisiertes Zirkoniumoxid-Kompositpulver mittels eines Co-Fällungsprozesses, wobei das Pulver sich durch eine hohe Bruchzähigkeit und Verschleißbeständigkeit auszeichnet.

Ein keramisches Material mit einer Gradientenstruktur, bestehend aus mehreren Schichten Zirkoniumoxid und einer Oberflächenschicht aus Hydroxylapatit, ist aus der CN 1709829 A bekannt. Dieses Material wird trockengepresst und anschließend drucklos bei 1500°C bis 1600°C gesintert, wobei ein keramisches Material entsteht, das als lasttragende Komponente in verschiedenen Teilen des menschlichen Körpers eingesetzt werden kann.

Ebenfalls ist aus der KR 101081687 A eine Beschichtung eines Zirkoniumoxidkörpers mit einer dicken Hydroxylapatitschicht bekannt, die zum Erhalt des Kompositmaterials angewandt wird.

Bekannt sind Verfahren zur Herstellung von keramischen Schäumen über Infiltration eines Polymerschaumes mit einer Suspension, bestehend aus dispergierten Keramikpartikeln in einer Flüssigkeit, wie z. B. Wasser DE 196 21 638 C2. Der Polymerschaum wird während des Sinterns ausgebrannt. Die Keramikpartikeln formen ein exaktes Abbild des Polymerschaumes. Keramische Schäume werden auch über CVD-Verfahren über Abscheidung auf netzförmigen Kohlenstoffschäumen erhalten.

In EP 1 117 626 B1 wird ein Verfahren zur Erzeugung von synthetischem Knochenersatzmaterial beschrieben, welches aus einer großporigen Schaumkeramik besteht, deren offene Schaumstruktur Poren mit einem modalen Durchmesser von 100 µm aufweist. Das Verfahren umfasst:
1. das Bilden eines keramischen Schlickers, der ein im Wesentlichen homogenes Gemisch aus einem aus Partikeln bestehenden keramischen Stoff, einem organischen Bindemittel in einem flüssigen Trägermaterial und wahlweise einem oder mehreren grenzflächenaktiven Stoffen enthält, wobei mindestens einer der grenzflächenaktiven Stoffe vorhanden ist, falls das organische Bindemittel nicht als grenzflächenaktiver Stoff fungiert;
2. Aufschäumen des keramischen Schlickers mit Hilfe einer Kugelmühle; und
3. Erwärmen des aufgeschäumten keramischen Schlickers auf eine Temperatur, die ausreicht, um das organische Bindemittel im Wesentlichen auszubrennen.

Ein anderes Verfahren gemäß WO 03/008357 A2 arbeitet mit einem anorganischen Material, einem Metallpulver oder einer Metallpaste, wobei in einer wässrigen keramischen Masse eine direkte Reaktion mit Gasbildung zwischen den Mineralen (z. B. Kaolinite) und dem Metallpulver (z. B. Aluminium) abläuft. Der Porenbildungsprozess findet bei Raumtemperatur oder bei höheren Temperaturen in etwa 2 bis 60 Minuten ab. Der entstandene poröse Grünling wird in der Form oder unter Einsatz von Mikrowellen getrocknet.

Ein weiteres bekanntes Verfahren zur Herstellung einer offenzelligen Schaumkeramik ist die Direktschäumung durch ein Treibmittel (Produktblatt Schäumungsmittel W 53 FL., Fa. Zschimmer & Schwarz GmbH & Co., Lahnstein). Dafür wird zunächst eine Suspension aus keramischen Partikeln und Wasser oder einem Lösungsmittel hergestellt. Zu dieser Suspension werden ein Treibmittel und Polymerkomponenten zugegeben. anschließend wird diese Suspension in eine Form gegossen und die Reaktion des Treibmittels gestartet. Durch diese Reaktion kommt es zur Entwicklung von Gasblasen, die zu einem Aufschäumen der Suspension führen. Anschließend werden die Polymerkomponenten vernetzt, wodurch der Schaum erstarrt. Die Polymerkomponenten werden danach ausgebrannt und der verbliebene Schaum wird gesintert.
Der Nachteil dieses Verfahrens besteht darin, dass die Schäumung schwierig zu steuern ist.

Ebenfalls bekannt ist ein Verfahren zur Herstellung von Schaumkeramik durch Direktschäumung mittels Luft WO 97/45381. Dabei wird in eine Suspension aus keramischen Partikeln und Wasser oder einem Lösungsmittel eine Polymerkomponente zugegeben. Anschließend werden Luftblasen in die Suspension durch einen schnell laufenden Spezialrührer eingebracht. Die schaumige Suspension wird dann in eine Form eingebracht und durch Vernetzung der Polymerkomponente erstarrt der Schaum. Anschließend wird die Polymerkomponente ausgebrannt und der Schaum gesintert.
Mittels dieses Verfahrens sind nur sehr feine Schäume herstellbar, die eine geringe Offenzelligkeit aufweisen. Die Festigkeit dieser Schäume ist begrenzt, da keine hohe Konzentration an Partikeln möglich ist, und beim Schwinden während des Sinterns ebenfalls Spannungen und Risse auftreten, die die Festigkeit der Schaumkeramik begrenzen. Außerdem ist die Durchströmbarkeit des Schaumes beeinträchtigt, da die Poren häufig geschlossene Zellen darstellen.

Weiterhin ist ein Verfahren zur Herstellung leichtgewichtiger Grün- und Formkörper aus keramischen und/oder pulvermetallurgischen Materialien bekannt (DE 10 2008 000 100 A1), bei dem eine Suspension aus keramischen und/oder pulvermetallurgischen Materialien und einem Suspensionsmedium hergestellt, die Suspension einem Unterdruck bis zur Verfestigung des Suspensionsmediums ausgesetzt und dann das Suspensionsmedium sublimiert und entfernt wird.

Nachteilig bei den bekannten Lösungen des Standes der Technik ist nach wie vor, dass Hydroxylapatit aufgrund seiner geringen mechanischen Festigkeit nicht als Dauerimplantat geeignet ist und seine Resorption durch den Körper immer noch verhältnismäßig lang dauert.
Die als Dauerimplantat verwendeten Titan- oder Stahllegierungsgerüste weisen zwar eine sehr gute mechanische Druckfestigkeit aus, jedoch kann es zu diversen Unverträglichkeitsreaktionen, wie Allergien des Probanden, durch in den Körper abgegebene metallische Ionen kommen.
Die als Dauerimplantat verwendeten Materialien, wie Zirkoniumoxid und Zirkoniumoxid-verstärktes Aluminiumoxid (ZTA), weisen ebenfalls eine sehr gute mechanische Festigkeit auf, sind aber nach wie vor dadurch benachteiligt, dass keine Resorption durch den menschlichen Körper möglich ist, da diese Materialien per se nicht bioaktiv sind und in der Regel dicht gesintert werden. Hinzu kommt, dass diese Materialien als synthetische Knochenersatzmaterialien eben aufgrund ihrer sehr hohen mechanischen Festigkeiten nicht geeignet sind, da sich das angrenzende Stützgewebe zurückbilden wird.

Aufgabe der vorliegenden Erfindung ist es daher, ein synthetisches Knochenersatzmaterial anzugeben, welches biokompatibel und gut resorbierbar (bioaktiv) ist, sowie einerseits offenzellig ist und andererseits eine gute mechanische Festigkeit aufweist, sowie ein einfaches und kostengünstiges Verfahren zu seiner Herstellung.

Die Aufgabe wird durch die in den Ansprüchen angegebene Erfindung gelöst. Vorteilhafte Ausgestaltungen sind Gegenstand der Unteransprüche.

Das erfindungsgemäße synthetische Knochenersatzmaterial besteht aus einem überwiegend offenzelligen Netzwerk aus dreidimensional miteinander verbundenen Stegen, die aus 20 - 40 Vol.-% eines oder mehrerer Calciumphosphate und aus 60 - 80 Vol.-% mindestens eines biokompatiblen keramischen Materials bestehen, wobei mindestens eine gebietsweise Perkolation des biokompatiblen keramischen Materials vorliegt, und wobei das offenzellige Netzwerk neben- und/oder übereinander und/oder ineinander verschlungen und/oder ineinander übergehende Bereiche von Calciumphosphaten und biokompatiblen keramischen Materialien aufweist, und das Netzwerk eine mehrmodale Verteilung aufweist, die einerseits aus den Zellen des Netzwerkes und andererseits aus den Poren in den Netzwerkmaterialien gebildet ist, und das Netzwerkmaterial eine maximale Porosität von 50 % aufweist.

Vorteilhafterweise sind als ein oder mehrere Calciumphosphate Hydroxylapatit, Octacalciumphosphat oder Tricalciumphosphat oder fremdionensubstituierte Calciumphosphate vorhanden.

Weiterhin vorteilhafterweise sind als biokompatibles keramisches Material Aluminiumoxid, Zirkoniumoxid, teilstabilisiertes Zirkoniumoxid oder zirkoniumverstärktes Aluminiumoxid vorhanden.

Ebenfalls vorteilhafterweise weist das offenzellige Netzwerk mindestens teilweise ineinander verschlungene und ineinander übergehende Bereiche von Calciumphosphaten und biokompatiblen keramischen Materialien auf.

Und auch vorteilhafterweise liegt eine bimodale Verteilung mit Maxima bei 0,1 bis 2 µm der Poren und bei 40 bis 2000 µm der Zellen vor.

Vorteilhaft ist es auch, wenn die Maxima im Bereich von 0,1 µm bis 10 mm vorhanden sind, vorteilhafterweise im Bereich von 1 µm bis 1 mm der Poren, oder vorteilhafterweise im Bereich von 10 µm bis 800 µm der Zellen und noch vorteilhafterweise im Bereich von 100 µm bis 800 µm.

Ebenfalls vorteilhaft ist es. wenn das Netzwerkmaterial eine maximale Porosität von 5 - 30 % aufweist.

Weiterhin vorteilhafterweise liegt die Perkolation des biokompatiblen keramischen Materials durch das gesamte synthetische Knochenmaterial vor.

Bei dem erfindungsgemäßen Verfahren zur Herstellung eines synthetischen Knochenersatzmaterials wird aus einem Pulver aus einem oder mehreren Calciumphosphaten und aus einem Pulver aus mindestens einem biokompatiblen keramischen Material jeweils mit einem Suspensionsmedium eine Suspension mit 20 - 40 Vol.-% an Calciumphosphaten und 60 - 80 Vol.-% an biokompatiblen keramischen Materialien hergestellt, die Suspension auf eine Unterlage gegeben und einem Unterdruck bis mindestens zur Verfestigung des Suspensionsmediums ausgesetzt wird, wobei vor der Verfestigung der Suspension auch eine oder mehrere weitere Suspensionen auf die Suspension aufgebracht und nachfolgend einem Unterdruck bis mindestens zur Verfestigung der Suspensionsmedium ausgesetzt werden, oder nach der Verfestigung einer oder mehrerer der Suspensionen eine oder mehrere Suspensionen auf die verfestigte Suspension(en) aufgebracht und einem Unterdruck bis mindestens zur Verfestigung der Suspensionsmedien ausgesetzt werden, und anschließend der Grünkörper aus dem verfestigten Suspensions-Netzwerk gesintert wird.

Vorteilhafterweise werden als Suspensionsmedium Wasser oder wasserhaltige Lösungsmittel, tertiäres Butanol, Cyclohexan oder Cyclohexanol eingesetzt.

Es ist auch von Vorteil, wenn eine Suspension in eine Form gegeben wird, diese einem Unterdruck bis mindestens zur Verfestigung des Suspensionsmediums ausgesetzt wird, nachfolgend auf und/oder über und/oder in die verfestigte Suspension eine oder mehrere weitere Suspensionen auf- und/oder eingebracht werden, wobei dabei mindestens eine der Suspensionen Calciumphosphate und mindestens eine der Suspensionen biokompatible keramische Materialien aufweist, und diese Suspensionen wiederum einem Unterdruck bis mindestens zur Verfestigung des Suspensionsmediums ausgesetzt werden.

Weiterhin ist von Vorteil, wenn beim Aufbringen mindestens einer Suspension auf eine oder mehrere bereits verfestigte Suspensionen mindestens eine der Suspensionen Calciumphosphate und mindestens eine der Suspensionen biokompatible keramische Materialien aufweist.

Ebenfalls ist es von Vorteil, wenn als Formenmaterialien zum Einfüllen der Suspension Silikonkautschuk, Metalle oder Eis verwendet werden.

Vorteilhaft ist es auch, wenn Suspensionen eingesetzt werden, die eine Temperatur zwischen 0 °C und 50 °C aufweisen.

Weiterhin vorteilhaft ist es, wenn Suspensionen eingesetzt werden, die Feststoffgehalte zwischen 20 und 60 Vol.-% aufweisen.

Und auch vorteilhaft ist es, wenn ein Suspensionsmedium eingesetzt wird, welches eine Schmelztemperatur > - 70 °C und < 25 °C aufweist und sich über eine Sublimationstrocknung entfernen lässt.

Es ist auch vorteilhaft, wenn die Suspension aus 35 - 40 Vol.-% an Calciumphosphaten und 60 - 65 Vol.-% an biokompatiblen keramischen Materialien hergestellt wird.

Mit der erfindungsgemäßen Lösung wird es erstmals möglich, ein synthetisches Knochenersatzmaterial anzugeben, welches biokompatibel und gut resorbierbar ist und gleichzeitig offenzellig ist und vergleichbare mechanische Festigkeit wie natürliches Knochenersatzmaterial aufweist.

Erreicht wird dies mit dem erfindungsgemäßen synthetischen Knochenersatzmaterial, welches aus einem offenzelligen Netzwerk aus dreidimensional miteinander verbundenen, vollen Stegen besteht. Dabei besteht das Netzwerk aus einem oder mehreren Calciumphosphaten und weiterhin aus mindestens einem biokompatiblen keramischen Material.

In dem Netzwerk können die Calciumphosphate und biokompatiblen keramischen Materialien neben- oder übereinander angeordnet sein, wobei innerhalb eines erfindungsgemäßen synthetischen Knochenersatzmaterials auch mehrere Bereiche aus einem Netzwerk aus Calciumphosphaten und mehrere Bereiche aus einem Netzwerk des biokompatiblen keramischen Materials bestehen können. In den Übergangsbereichen können die Netzwerke ohne stoffliche Verbindung nebeneinander vorliegen, oder sie können auch ganz oder teilweise eine stoffliche Verbindung aufweisen.
Die erfindungsgemäßen Netzwerke können aber auch ganz oder teilweise ineinander verschlungen vorliegen und/oder ineinander übergehende Bereiche von Calciumphosphaten und biokompatiblen keramischen Materialien aufweisen. Insbesondere möglichst vollständig ineinander verschlungene Netzwerke mit ineinander übergehenden Bereichen aus bioaktiven Calciumphosphaten und biokompatiblen keramischen Materialien sind vorteilhaft, da sie die mechanische Festigkeit des keramischen Materials mit der Bioaktivität der Calciumphosphate vereinen. Dabei wird durch das Vorhandensein von 20 - 40 Vol.-% an Calciumphosphaten und 60 - 80 Vol.-% an biokompatiblen keramischen Materialien die Perkolationsschwelle für die biokompatiblen keramischen Materialien überschritten, so dass die Bereiche an biokompatiblen keramischen Materialien die Bereiche an Calciumphosphaten möglichst vollständig umschließen und durchgehend miteinander verbunden sind. Die Perkolation des biokompatiblen keramischen Materials liegt in jedem Fall bereichsweise im synthetischen Knochenersatzmaterial vor, vorteilhafterweise im gesamten synthetischen Knochenersatzmaterial. Dadurch wird die erhöhte mechanische Festigkeit im erfindungsgemäßen synthetischen Knochenersatzmaterial erreicht.

Die erfindungsgemäßen Netzwerke aus Calciumphosphaten und biokompatiblen keramischen Materialien weisen weiterhin eine mehrmodale Verteilung auf, wobei mindestens ein Maximum von den Zellen des Netzwerkes und andererseits ein Maximum von den Poren in den Netzwerkmaterialien gebildet wird. Dabei darf die Porosität der Netzwerkmaterialien maximal 50 % betragen.

Unter den erfindungsgemäßen Netzwerken aus Calciumphosphaten und biokompatiblen keramischen Materialien soll im Rahmen dieser Erfindung ein Netzwerk verstanden werden, welches Zellen aufweist, die von Stegen gebildet werden. Dabei sind die Zellen durch das Treibmittel während des Schäumens entstanden. Die Stege bestehen erfindungsgemäß aus Calciumphosphaten und aus biokompatiblen keramischen Materialien, wobei beim Grünkörper noch Bindemittel und/oder Hilfs- und Zusatzstoffe für die Sinterung der Materialien vorhanden sein können. Die Zellen sind überwiegend offen, das heißt die Zellfenster sind geöffnet. Es können erfindungsgemäß aber auch geschlossene Zellen vorliegen. Diese Zellen des Netzwerkes sind nicht zu verwechseln mit den biologischen Zellen, die auf den Netzwerken angesiedelt werden, um dort zu wachsen.

Im Unterschied zu den Zellen des Netzwerkes können auch in den Stegen offene und geschlossene Poren vorliegen, sowohl vor als auch nach der Sinterung. Diese Poren in den Stegen sind von den Zellen des Netzwerkes zu unterscheiden. Die Porosität der Netzwerkmaterialien in den Stegen beträgt erfindungsgemäß maximal 50 %.

Die erfindungsgemäßen Netzwerke bestehen aus einem geschäumten oder porösen Körper mit einer spezifischen Masse von 10 bis 90 % des reinen Materials mit einer Vielzahl von offenen, teilweise miteinander verbundenen, im allgemeinen ellipsoide Zellen, wobei auch eine geringe Anzahl an geschlossenen Zellen vorliegen kann. Die Zellen des Netzwerkes und die Poren der Stege weisen insgesamt eine mehrmodale Verteilung auf, wobei vorteilhafterweise eine bimodale Verteilung vorliegt. Die mehrmodale Verteilung bezieht sich auf die Gesamtheit der Porosität der Schaumstruktur - die der Zellen und die der Poren in den Stegen.
Die Maxima einer biomodalen Verteilung liegen vorteilhafterweise bei 0,1 bis 2 µm für die Poren und bei 40 bis 2000 µm für die Zellen.
Die Verteilung liegt insgesamt vorteilhafterweise im Bereich von 0,1 µm bis 10 mm, vorteilhafterweise im Bereich von 1 µm bis 1 mm, vorteilhafterweise im Bereich von 10 µm bis 800 µm, vorteilhafterweise im Bereich von 100 µm bis 800 µm.

Gegenüber metallischen Schäumen, die über Schmelze-Verfahren erzeugt wurden, unterscheiden sich die erfindungsgemäßen Netzwerke dadurch, dass sie über eine Sinterung ihre Endfestigkeit erhalten. Während die metallischen Schäume ein Erstarrungsgefüge aufweisen, zeigen die erfindungsgemäßen Strukturen der Formkörper ein materialtypisches Sintergefüge.

Gegenüber keramischen und metallischen Schaumstrukturen, die über Beschichten polymerer Schaumskelettstrukturen mit Pulversuspensionen erzeugt werden, zeichnen sich die erfindungsgemäßen Strukturen der Grün- und Formkörper dadurch aus, dass sie keine Hohlstege aufweisen, welche Replikate der ausgebrannten Polymerstrukturen darstellen.

Die Strukturen der erfindungsgemäßen Grünkörper lassen sich von allen anderen keramischen und metallischen Schaumstrukturen dadurch unterscheiden, dass die Stege der Grünkörper gefüllt sind und Oberflächenmerkmale sowie Poren aufweisen, die von der Erstarrung des Suspensionsmediums und dem häufig damit verbundenen Kristallwachstum herrühren. Diese charakteristischen Erstarrungsstrukturen lassen sich beispielsweise im Rasterelektronenmikroskop nachweisen und geben einen Hinweis auf die Entstehungsgeschichte der jeweiligen geschäumten, porösen Struktur.

Hergestellt wird das erfindungsgemäße synthetische Knochenersatzmaterial indem aus einem Pulver aus einem oder mehreren Calciumphosphaten und aus einem Pulver aus mindestens einem biokompatiblen keramischen Material mit einem Suspensionsmedium eine Suspension mit 20 - 40 Vol.-% an Calciumphosphaten und 60 - 80 Vol.-% an biokompatiblen keramischen Materialien hergestellt wird. Calciumphosphate sollen erfindungsgemäß im Unterschied zu den weiteren biokompatiblen keramischen Materialien bioaktiv sein und weisen gegenüber den weiteren biokompatiblen keramischen Materialien eine geringere mechanische Festigkeit auf.

Aus den Calciumphosphaten und keramischen Materialien wird mit einem Suspensionsmedium und gegebenenfalls Bindemitteln und/oder Hilfs- oder Zusatzstoffen für die Sinterung der Materialien eine Suspension mit Feststoffgehalten von > 10 bis 55 Vol.-% hergestellt.
Eine derartige erfindungsgemäß eingesetzte Suspension kann aufgrund der Feststoffgehalte eine Viskosität von leicht flüssig bis pastös aufweisen.

Als Suspensionsmedium können Wasser oder ein wasserhaltiges Lösungsmittel eingesetzt werden.

Die Suspension kann vorteilhafterweise gerührt werden.

Über die Menge an eingebrachter Luft oder anderen Gasen in die Suspension und die Viskosität der Suspension kann die Anzahl und Größe der Zellen, die durch die Stege gebildet werden, gesteuert werden.

Nachfolgend wird eine der Suspensionen auf eine Unterlage gegeben. Diese Unterlage kann auch eine Form sein, die den entstehenden Grünköper gleich formt.

Die Suspension wird dann einem Unterdruck bis mindestens zur Verfestigung des Suspensionsmediums ausgesetzt.
Durch das erfindungsgemäße Verfahren wird die Suspension, während sie einem Unterdruck bis mindestens zur Verfestigung des Suspensionsmediums ausgesetzt sind, aufgeschäumt. Durch das Anlegen des Unterdruckes werden die in der Suspension zwangsläufig vorhandene Luft oder andere Gase zum Treibmittel für den Schaumbildungsprozess. Dadurch und durch den aufgrund des verringerten Umgebungsdruckes abgesenkten Siedepunkt des Suspensionsmediums schäumt die Suspension auf und es entsteht ein offenzelliges Netzwerk als hochporöse Struktur bis zu einer Schaumstruktur.

Erfindungsgemäß kann auch vor der Verfestigung der ersten Suspension eine oder mehrere weitere Suspensionen auf die Suspension aufgebracht und nachfolgend einem Unterdruck bis mindestens zur Verfestigung der Suspensionsmedien ausgesetzt werden. Ebenfalls ist es erfindungsgemäß, wenn nach der Verfestigung einer oder mehrerer der Suspensionen eine oder mehrere Suspensionen auf die verfestigte Suspension(en) aufgebracht und einem Unterdruck bis mindestens zur Verfestigung der Suspensionsmedium ausgesetzt werden.

Erfindungsgemäß erforderlich ist, dass mindestens die Suspension aus einem oder mehreren Calciumphosphaten und biokompatiblen keramischen Materialien mindestens einmal aufgebracht und verfestigt wird.
Nach der Herstellung des Netzwerkes wird die Sublimation des Suspensionsmediums durchgeführt und der Grünkörper hergestellt.

Anschließend wird der Grünkörper aus einem verfestigten Suspensions-Netzwerk erfindungsgemäß gesintert. Die Sinterung wird in einem für die Verdichtung von

Calciumphosphaten und insbesondere von Hydroxylapatit relevanten Temperaturbereich von 1250 - 1400 °C durchgeführt.

Die dabei entstandene Sinterstruktur ist mechanisch stabil und bearbeitbar und besonders druckfest.
Die Netzwerk- und Zellstruktur des erfindungsgemäßen synthetischen Knochenersatzmaterials ist hinsichtlich seiner Zellgeometrie und -größe besonders geeignet, um ein schnelles Zellwachstum für biologische Zellen zu ermöglichen.

Besonders vorteilhaft ist es, wenn erfindungsgemäß eine Suspension auf eine Unterlage gegeben wird, diese einem Unterdruck bis mindestens zur Verfestigung des Suspensionsmediums ausgesetzt wird, nachfolgend auf und/oder über und/oder in die verfestigte Suspension eine oder mehrere weitere Suspensionen auf- und/oder eingebracht werden, wobei dabei mindestens eine der Suspensionen Calciumphosphate und biokompatible keramische Materialien aufweist, und diese Suspensionen wiederum einem Unterdruck bis mindestens zur Verfestigung des Suspensionsmediums ausgesetzt werden. Dadurch entsteht ein ineinander verschlungenes Netzwerk mit ineinander übergehenden Bereichen aus Calciumphosphaten und keramischen Materialien, welches besonders gut resorbierbar ist.

Weiterhin können den Suspensionen neben den Calciumphosphaten und den keramischen Materialien auch weitere beispielsweise faserförmige oder plättchenförmige Materialien hinzugefügt werden.
Die Fasern können Lang- oder Kurzfasern sein und aus Kohlenstoff, Biogläsern, oxidisch bioinerten Materialien, wie Aluminiumoxid, Zirkoniumoxid oder ZTA, oder aus bioinerten Metallen, wie Edelstahl oder Titan, bestehen.

Die eingesetzten biokompatiblen keramischen Materialien werden insbesondere auch danach ausgewählt, dass ihre Endfestigkeit im Netzwerk angenähert ist der Festigkeit von natürlichen Knochenersatzmaterialien.

Ein besonderer Vorteil des erfindungsgemäßen Verfahrens besteht darin, dass die Suspensionen in Formen gegeben werden können und der Schäumungsprozess so gesteuert werden kann, dass ein endformnaher Grünkörper hergestellt werden kann, bei dem die Schwindung bei der Sinterung berücksichtigt worden ist und damit ein patientenspezifisch angepasstes synthetisches Knochenersatzmaterial hinsichtlich seiner Form und Abmessungen herstellbar ist. Eine mechanische Nachbearbeitung ist möglich, kann aber auch aufgrund der gewählten Verfahrensführung entfallen. Als Formen können Silikonkautschukformen, Eis- oder Metallformen eingesetzt werden.

Die Vorteile der vorliegenden Erfindung liegen in der Angabe eines biokompatiblen und bioaktiven Knochenersatzmaterials, welches einen hohen Anteil an offenen Zellen aufweist und damit ein sehr gutes Einwachsen der biologischen Zellen ermöglicht. Damit ist das erfindungsgemäße Knochenersatzmaterial sehr gut resorbierbar. Dadurch dass die Stege der Netzwerke im Wesentlichen vollständig aus Calciumphosphaten und biokompatiblen keramischen Materialien bestehen und nur eine geringe Porosität von maximal 50 % aufweisen, weisen die Netzwerke eine hohe Druckfestigkeit auf, die dem gesamten Knochenersatzmaterial zu Gute kommt. Dieser Vorteil wird insbesondere sichtbar, wenn das Knochenersatzmaterial aus einander durchdrungenen Netzwerken aus Calciumphosphat und keramischen Materialien besteht.

Nachfolgend wird die Erfindung an mehreren Ausführungsbeispielen näher erläutert.

### Beispiel 1

350 g Aluminiumoxidpulver (TM-DAR, Taimai) mit einer Teilchengrößenverteilung von d50 = 0,15 µm werden in 30 g Wasser unter Zugabe von 4 ml eines Verflüssigers (Dolapix CE64 Zschimmer & Schwarz) unter Rühren im Ultraschallbad dispergiert. Als temporärer Binder werden der Suspension 10 g Polyvinylalkohol (PVA)-Lösung zugegeben. Gegen Ende erfolgt das Zugeben 4 g Assoziativverdickers (Tafigel PUR, Münzing Chemie). 50 g der Suspension mit einem Feststoffgehalt von ca. 90 Ma.-% werden nach 1 h Rühren bei Raumtemperatur in eine zylindrische Silikonkautschukform (Durchmesser 25 mm) eingefüllt und auf der Stellfläche eines Gefriertrockners (Gamma 20, Fa. Martin Christ) positioniert. Anschließend wird der Druck in der Vakuumkammer des Gefriertrockners auf einen Druck von 125 Pa abgesenkt, was einer Gleichgewichtstemperatur des Eises von -18 °C entspricht. Während der Druckabsenkung wird die Suspension aufgeschäumt. Bei Erreichen des Gefrierpunktes der Suspension friert der so entstandene Schaum schlagartig ein. Das gefrorene Suspensionsmedium wird über Sublimationstrocknung entfernt.

Nunmehr wird aus 40 g Hydroxylapatit (Fa. SIGMA-ALDRICH) mit einem Teilchengrößenverteilung von d50 = 2,64 µm und 20 ml Wasser, versetzt mit 1 ml Verflüssiger (Dolapix) und 10 g PVA-Binder, eine weitere Suspension, ebenfalls unter Rühren im Ultraschallbad, erzeugt. Gegen Ende erfolgt wieder die Zugabe 4 g Assoziativverdickers.
50 g Suspension wird über den getrockneten Schaum aus Aluminiumoxid gefüllt. Dieser gefüllte Schaum wird dann wieder auf der Stellfläche eines Gefriertrockners (Gamma 20, Fa. Martin Christ) positioniert. Anschließend wird der Druck in der Vakuumkammer des Gefriertrockners auf einen Druck von 125 Pa abgesenkt, was einer Gleichgewichtstemperatur des Eises von -18 °C entspricht. Während der Druckabsenkung wird die Hydroxylapatit-Suspension aufgeschäumt. Bei Erreichen des Gefrierpunktes der Suspension friert der so entstandene Schaum wiederum schlagartig ein.

Nach Erreichen des Vakuumdruckes von 125 Pa wird die Stellflächenheizung auf 30 °C eingestellt und die Gefriertrocknung gestartet. Nach 48 h wird der Gefriertrocknungsprozess beendet und die getrocknete Schaumprobe als Grünkörper entnommen. Die Entfernung des temporären Bindemittels erfolgt bei 500 °C. Nachfolgend wird die Sinterung an Luft mit einer Aufheizrate von 3 K/min bei 1350 °C über 1 h Haltezeit durchgeführt.
Nach der Sublimationstrocknung, dem Entbindern und Sintern, liegt ein druckfester, biokompatibler Schaum mit einer bioaktiven, durchdringenden/belegten Schaumstrukturkomponente vor.
Die auf diese Weise erhaltene geschäumte Struktur weist eine Dichte der Stege von 56 % der theoretischen Dichte von Aluminiumoxid (3,99 g/cm³) auf. Der Anteil an offenen Zellen zwischen den Stegen beträgt 80%.

### Beispiel 2

50 g Zirkoniumdioxid (TZ-3Y-E, Fa. TOSOH) mit einer Teilchengrößenverteilung von d50 = 0,73 µm werden in 25 g Wasser unter Zugabe von 1 ml eines Verflüssigers (Dolapix CE64 Zschimmer & Schwarz) unter Rühren im Ultraschallbad dispergiert. Als temporärer Binder werden der Suspension 3 g PVA-Lösung zugegeben. 50 g der Suspension mit einem Feststoffgehalt von ca. 64 Ma.-% werden nach 1 h Rühren bei Raumtemperatur in eine zylindrische Form (Durchmesser 40 mm) eingefüllt.

Nunmehr wird aus 40 g Hydroxylapatit (Fa. SIGMA-ALDRICH) mit einer Teilchengrößenverteilung von d50 = 2,64 µm und 20 ml Wasser, versetzt mit 2 ml Verflüssiger (Dolapix) und 10 g PVA-Binder, eine weitere Suspension, ebenfalls unter Rühren im Ultraschallbad, erzeugt. Gegen Ende erfolgt hier die Zugabe von 5 g Assoziativverdickers. Die Hydroxylapatit Suspension wird nun in die gleiche Form gefüllt, in der bereits die ZrO₂-Suspension gegeben wurde.

Nun werden nochmals 50 g der ZrO₂-Suspension als schwerere Komponente (entsprechend Dichte) auf die Hydroxylapatit-Suspension aufgeschichtet. Ebenso können die Suspensionen auch in umgekehrter Reihenfolge in die Form gegeben werden oder auch nur zwei oder mehr als drei.

Die Form mit den drei aufgeschichteten Suspensionen wird schließlich in den Gefriertrockner überführt und das Verfahren gemäß Beispiel 1 realisiert. Durch die Absenkung des Druckes beginnen die Suspensionen aufzuschäumen. An den Grenzflächen der Schichten kommt es nun zu einer Überlappung der verschiedenen Materialien. Dabei können sich diese verschränken und mindestens teilweise durchdringen. Nach der Sublimationstrocknung werden die mindestens teilweise ineinander verschlungenen Schäume entbindert und gesintert.

### Beispiel 3

Es wird eine Suspension aus ZrO₂ hergestellt (gemäß Beispiel 2), in den Gefriertrockner überführt, dort aufgeschäumt und der entstandene Schaum durch Sublimation des Suspensionsmediums getrocknet. Anschließend erfolgt die Entbinderung und Sinterung.
Nunmehr wird diese gesinterte, poröse Struktur genommen und in eine neu angefertigte Tricalciumphosphatsuspension (hergestellt wie in Beispiel 1), welche sich in einer Form befindet, gelegt. Diese beiden Werkstoffe werden wiederum in dem Gefriertrockner überführt und nun die flüssige bioaktive Tricalciumphosphatsuspension um den gesinterten ZrO₂-Schaum herum, analog zu den oben genannten Beispielen, aufgeschäumt. Nach der Sublimationstrocknung, dem Entbindern und Sintern, liegt ein druckfester, biokompatibler Schaum mit einer bioaktiven, durchdringenden/belegten Schaumstrukturkomponente vor.

## Patentansprüche

1. Synthetisches Knochenersatzmaterial bestehend aus einem überwiegend offenzelligen Netzwerk aus dreidimensional miteinander verbundenen Stegen, die aus 20 - 40 Vol.-% eines oder mehrerer Calciumphosphate und aus 60 - 80 Vol.-% mindestens eines biokompatiblen keramischen Materials bestehen, wobei mindestens eine gebietsweise Perkolation des biokompatiblen keramischen Materials vorliegt, und wobei das offenzellige Netzwerk neben-und/oder übereinander und/oder ineinander verschlungene und/oder ineinander übergehende Bereiche von Calciumphosphaten und biokompatiblen keramischen Materialien aufweist, und das Netzwerk eine mehrmodale Verteilung aufweist, die einerseits aus den Zellen des Netzwerkes und andererseits aus den Poren in den Netzwerkmaterialien gebildet ist, und das Netzwerkmaterial eine maximale Porosität von 50 % aufweist.

2. Synthetisches Knochenersatzmaterial nach Anspruch 1, bei dem als ein oder mehrere Calciumphosphate Hydroxylapatit, Octacalciumphosphat oder Tricalciumphosphat oder fremdionensubstituierte Calciumphosphate vorhanden sind.

3. Synthetisches Knochenersatzmaterial nach Anspruch 1, bei dem als biokompatibles keramisches Material Aluminiumoxid, Zirkoniumoxid, teilstabilisiertes Zirkoniumoxid oder zirkoniumverstärktes Aluminiumoxid vorhanden sind.

4. Synthetisches Knochenersatzmaterial nach Anspruch 1, bei dem das offenzellige Netzwerk mindestens teilweise ineinander verschlungene und ineinander übergehende Bereiche von Calciumphosphaten und biokompatiblen keramischen Materialien aufweist.

5. Synthetisches Knochenersatzmaterial nach Anspruch 1, bei dem eine bimodale Verteilung mit Maxima bei 0,1 bis 2 µm der Poren und bei 40 bis 2000 µm der Zellen vorliegt.

6. Synthetisches Knochenersatzmaterial nach Anspruch 1, bei dem Maxima im Bereich von 0,1 µm bis 10 mm vorhanden sind.

7. Synthetisches Knochenersatzmaterial nach Anspruch 6, bei dem Maxima der Poren im Bereich von 1 µm bis 1 mm vorhanden sind.

8. Synthetisches Knochenersatzmaterial nach Anspruch 6, bei dem Maxima der Zellen im Bereich von 10 µm bis 800 µm vorhanden sind.

9. Synthetisches Knochenersatzmaterial nach Anspruch 8, bei dem Maxima der Zellen im Bereich von 100 µm bis 800 µm vorhanden sind.

10. Synthetisches Knochenersatzmaterial nach Anspruch 1, bei dem das Netzwerkmaterial eine maximale Porosität von 5 - 30 % aufweist.

11. Synthetisches Knochenmaterial nach Anspruch 1, bei dem die Perkolation des biokompatiblen keramischen Materials durch das gesamte synthetische Knochenmaterial vorliegt.

12. Verfahren zur Herstellung eines synthetischen Knochenersatzmaterials, bei dem aus einem Pulver aus einem oder mehreren Calciumphosphaten und aus einem Pulver aus mindestens einem biokompatiblen keramischen Material jeweils mit einem Suspensionsmedium eine Suspension mit 20 - 40 Vol.-% an Calciumphosphaten und 60 - 80 Vol.-% an biokompatiblen keramischen Materialien hergestellt wird, die Suspension auf eine Unterlage gegeben und einem Unterdruck bis mindestens zur Verfestigung des Suspensionsmediums ausgesetzt wird, wobei vor der Verfestigung der Suspension auch eine oder mehrere weitere Suspensionen auf die Suspension aufgebracht und nachfolgend einem Unterdruck bis mindestens zur Verfestigung der Suspensionsmedium ausgesetzt werden, oder nach der Verfestigung einer oder mehrerer der Suspensionen eine oder mehrere Suspensionen auf die verfestigte Suspension(en) aufgebracht und einem Unterdruck bis mindestens zur Verfestigung der Suspensionsmedien ausgesetzt werden, und anschließend der Grünkörper aus dem verfestigten Suspensions-Netzwerk gesintert wird.

13. Verfahren nach Anspruch 12, bei dem als Suspensionsmedium Wasser oder wasserhaltige Lösungsmittel, tertiäres Butanol, Cyclohexan oder Cyclohexanol eingesetzt werden.

14. Verfahren nach Anspruch 12, bei dem eine Suspension in eine Form gegeben wird, diese einem Unterdruck bis mindestens zur Verfestigung des Suspensionsmediums ausgesetzt wird, nachfolgend auf und/oder über und/oder in die verfestigte Suspension eine oder mehrere weitere Suspensionen auf- und/oder eingebracht werden, wobei dabei mindestens eine der Suspensionen Calciumphosphate und mindestens eine der Suspensionen biokompatible keramische Materialien aufweist, und diese Suspensionen wiederum einem Unterdruck bis mindestens zur Verfestigung des Suspensionsmediums ausgesetzt werden.

15. Verfahren nach Anspruch 14, bei dem beim Aufbringen mindestens einer Suspension auf eine oder mehrere bereits verfestigte Suspensionen mindestens eine der Suspensionen Calciumphosphate und mindestens eine der Suspensionen biokompatible keramische Materialien aufweist.

16. Verfahren nach Anspruch 12, bei dem als Formenmaterialien zum Einfüllen der Suspension Silikonkautschuk, Metalle oder Eis verwendet werden.

17. Verfahren nach Anspruch 12, bei dem Suspensionen eingesetzt werden, die eine Temperatur zwischen 0 °C und 50 °C aufweisen.

18. Verfahren nach Anspruch 12, bei dem Suspensionen eingesetzt werden, die Feststoffgehalte zwischen 20 und 60 Vol.-% aufweisen.

19. Verfahren nach Anspruch 12, bei dem ein Suspensionsmedium eingesetzt wird, welches eine Schmelztemperatur > - 70 °C und < 25 °C aufweist und sich über eine Sublimationstrocknung entfernen lässt.

20. Verfahren nach Anspruch 12, bei dem eine Suspension mit 35 - 40 Vol.-% an Calciumphosphaten und 60 - 65 Vol.-% an biokompatiblen keramischen Materialien hergestellt wird.
